# EUROPÄISCHE PATENTSCHRIFT

(11) **EP 1 405 079 B1**
(45) Veröffentlichungstag und Bekanntmachung des Hinweises auf die Patenterteilung: **30.05.2012**
(21) Anmeldenummer: 01994703.5
(22) Anmeldetag: 22.11.2001
(51) Int. Cl.: G01N 33/68, G01N 33/542, G01N 33/574, C07K 16/18

(54) **MODULIERUNG DER WECHSELWIRKUNG ZWISCHEN EVH1-DOMÄNEN**
MODULATION OF THE INTERACTION BETWEEN EVH1 DOMAINS
MODULATION DE L'INTERACTION ENTRE DES DOMAINES EVH1

(30) Priorität: 25.11.2000 DE 10058596
(43) Veröffentlichungstag der Anmeldung: 07.04.2004
(73) Patentinhaber: Sanofi-Aventis Deutschland GmbH, 65929 Frankfurt am Main (DE); Vasopharm Biotech GmbH, 97082 Würzburg (DE)
(72) Erfinder: JORDAN, Birgit, 65795 Hattersheim (DE); DRÜCKES, Peter, 79100 Freiburg (DE); JARCHAU, Thomas, 97078 Würzburg (DE); WALTER, Ulrich, 97209 Veitshöchheim (DE)
(86) Internationale Anmeldenummer: PCT/EP2001/013592
(87) Internationale Veröffentlichungsnummer: WO 2002/042777

(56) Entgegenhaltungen:
- WO-A-01/74858
- WO-A-01/96594
- WO-A-98/01755
- WO-A1-97/29373
- WO-A2-98/15830
- US-A- 5 935 995
- DATABASE BIOSIS [Online] BIOSCIENCES INFORMATION SERVICE, PHILADELPHIA, PA, US; August 2001 (2001-08) BADER BENJAMIN ET AL: "A cGMP-dependent protein kinase assay for high throughput screening based on time-resolved fluorescence resonance energy transfer." Database accession no. PREV200100428261 XP002231552 & JOURNAL OF BIOMOLECULAR SCREENING, Bd. 6, Nr. 4, August 2001 (2001-08), Seiten 255-264, ISSN: 1087-0571
- NIEBUHR K ET AL: "A novel proline-rich motif present in ActA of Listeria monocytogenes and cytoskeletal proteins is the ligand for the EVH1 domain, a protein module present in the Ena/VASP family", EMBO JOURNAL, OXFORD UNIVERSITY PRESS, SURREY, GB, vol. 16, no. 17, 1 January 1997 (1997-01-01), pages 5433-5444, XP002183151, ISSN: 0261-4189
- REINHARD M ET AL: "VASP interaction with vinculin: a recurring theme of interactions with proline-rich motifs", FEBS LETTERS, ELSEVIER, AMSTERDAM, NL, vol. 399, no. 1/2, 9 December 1996 (1996-12-09), pages 103-107, XP007909147, ISSN: 0014-5793
- AHERN-DJAMALI S M ET AL: "Mutations in Drosophila enabled and rescue by human vasodilator-stimulated phosphoprotein (VASP) indicate important functional roles for Ena/VASP homology domain 1 (EVH1) and EVH2 domains", MOLECULAR BIOLOGY OF THE CELL, BETHESDA, MD, US, vol. 9, no. 8, 1 August 1998 (1998-08-01), pages 2157-2171, XP007909148, ISSN: 1059-1524
- BALL L J ET AL: "Dual epitope recognition by the VASP EVH1 domain modulates polyproline ligand specificity and binding affinity", EMBO JOURNAL, OXFORD UNIVERSITY PRESS, SURREY, GB, vol. 19, no. 18, 15 September 2000 (2000-09-15), pages 4903-4914, XP007909151, ISSN: 0261-4189
- MACALMA TERESITA ET AL: "Molecular characterization of human zyxin", JOURNAL OF BIOLOGICAL CHEMISTRY, AMERICAN SOCIETY OF BIOLOCHEMICAL BIOLOGISTS, BIRMINGHAM, US, vol. 271, no. 49, 1 January 1996 (1996-01-01), pages 31470-31478, XP002176724, ISSN: 0021-9258
- KRAUSE MATTHIAS ET AL: "Fyn-binding protein (Fyb)/SLP-76-associated protein (SLAP), Ena/vasodilator-stimulated phosphoprotein (VASP) proteins and the Arp2/3 complex link T cell receptor (TCR) signaling to the actin cytoskeleton", THE JOURNAL OF CELL BIOLOGY, ROCKEFELLER UNIVERSITY PRESS, US, vol. 149, no. 1, 3 April 2000 (2000-04-03) , pages 181-194, XP002188277, ISSN: 0021-9525
- SMOLENSKI A ET AL: "Regulation of human endothelial cell focal adhesion sites and migration by cGMP-dependent protein kinase I", JOURNAL OF BIOLOGICAL CHEMISTRY, AMERICAN SOCIETY OF BIOLOCHEMICAL BIOLOGISTS, BIRMINGHAM, US, vol. 275, no. 33, 20 August 2000 (2000-08-20), pages 25723-25732, XP007909206, ISSN: 0021-9258
- HOFFMAN LAURA M ET AL: "Genetic ablation of zyxin causes Mena/VASP mislocalization, increased motility, and deficits in actin remodeling.", THE JOURNAL OF CELL BIOLOGY 27 FEB 2006 LNKD- PUBMED:16505170, vol. 172, no. 5, 27 February 2006 (2006-02-27), pages 771-782, XPXP007914546, ISSN: 0021-9525
- HENES JANEK ET AL: "Inflammation-associated repression of vasodilator-stimulated phosphoprotein (VASP) reduces alveolar-capillary barrier function during acute lung injury.", THE FASEB JOURNAL : OFFICIAL PUBLICATION OF THE FEDERATION OF AMERICAN SOCIETIES FOR EXPERIMENTAL BIOLOGY DEC 2009 LNKD- PUBMED:19690214, vol. 23, no. 12, December 2009 (2009-12), pages 4244-4255, XPXP007914547, ISSN: 1530-6860
- HIROAKI HIRATA, HITOSHI TATSUMI, MASAHIRO SOKABE: "Zyxin emerges as a key player in the mechanotransduction at cell adhesive structures", COMMUNICATIVE & INTEGRATIVE BIOLOGY, vol. 1, no. 2, October 2008 (2008-10), pages 192-195, XP007914548,
- BADER B. ET AL: "A cGMP-dependent protein kinase assay for high throughput screening based on time-resolved fluorescence resonance energy transfer", JOURNAL OF BIOMOLECULAR SCREENING, vol. 6, no. 4, August 2001 (2001-08), pages 255-264, XP009006017, UNITED STATES

## Beschreibung

Verfahren zur Identifizierung einer chemischen Verbindung, welche die Wechselwirkung eines Proteins mit einer EVH1-Domäne und eines Proteins mit einer EVH1-Bindedomäne insbesondere zwischen VASP und Zyxin oder einem Zyxinderivat modulieren kann, sowie ein Verfahren zum Nachweis dieser Wechselwirkung werden beschrieben. Die Erfindung wird durch die Ansprüche definiert.

Die Wechselwirkung von Proteinen mittels EVH1-Domänen und EVH1-Bindedomänen spielt eine bedeutende Rolle insbesondere bei Signaltransduktionswegen, die beteiligt sind bei der Adhäsion von Zellen an Gewebeoberflächen und deren Motilität, der Änderung der Gestalt von Zellen und ihrer Aggregation insbesondere der Aktivierung von Blutplättchen und Lymphozyten. Solche Vorgänge wirken möglicherweise bei der Entstehung und dem Verlauf einer Vielzahl von Krankheiten ursächlich mit, insbesondere beispielsweise bei inflammatorischen Erkrankungen, Erkrankungen der Blutgefäße, des Herzkreislaufsystems und seiner Organe oder bei neoplastischen Zell- und Gewebsveränderungen wie Krebs.

Es gibt eine Vielzahl von Proteinen, welche eine EVH1-Domäne oder eine EVH1-Bindedomäne enthalten. Ein Protein mit einer EVH1-Bindedomäne ist beispielsweise Zyxin, während VASP beispielsweise ein Protein mit einer EVH1-Domäne ist. Die Domäne eines Proteins ist ein dreidimensionaler Bereich eines Proteins oder eine Proteinoberfläche, die von mehreren Abschnitten einer Peptidkette gebildet werden kann und sich als kompakter Bereich eigenständig falten kann. Einer EVH1(Ena-VASP-Homologie)-Domäne liegt ein hochkonservierter Sequenzabschnitt von etwa 115 Aminosäuren Länge zugrunde, der in allen Proteinen der Ena-VASP-Familie vorkommt und für ihre korrekte subzelluläre Lokalisation durch Wechselwirkung mit den jeweiligen EVH1-Bindeproteinen verantwortlich ist. Die EVH1-Domäne besteht sieben β-Faltblättern und einer C-terminalen α-Helix, die in einer charakteristischen "β-barrel"-Struktur gefaltet sind. Sie weist eine hohe strukturelle Ähnlichkeit zu Pleckstrin-Homologie(PH)- und zu Phosphotyrosin-Bindungs(PTB)-Domänen auf. Die EVH1-Domänen der Ena/VASP-Proteinfamilie erkennen in den verschiedenen EVH1-Bindeproteinen prolinreiche Peptidsequenzen mit einem FPPPP-Kernmotiv, die in Form einer Polyprolin-Helix vom Typ II gefaltet sind. EVH1-Bindeproteine mit solchen FPPPP-Sequenzmotiven sind z.B. die zytoskelettassozierten Proteine Zyxin und Vinculin oder das Oberflächenprotein ActA des fakultativ intrazellulären Bakteriums Listeria monocytogenes. Zyxin und VASP wechselwirken aufgrund der Interaktion der EVH1-Domänen von VASP und der FPPPP-Motive in der EVH1-Bindedomäne von Zyxin. VASP steht als Abkürzung für "Vasodilator-Stimulated Phosphoprotein". VASP wird in fast allen Säugerzellen exprimiert und stellt dort ein Substrat der cAMP- und cGMP-abhängigen Proteinkinasen dar. Homologe Proteine zu VASP bilden zusammen mit letzterem die Ena/VASP-Proteinfamile und konnten wie z.B. das Ena-Protein in Drosophila oder die Mena- und Evl-Proteine in der Maus nachgewiesen werden. Besonders hohe Konzentrationen an VASP finden sich beim Menschen in kardiovaskulären Zellen, insbesondere in Thrombozyten, Endothelzellen, in glatten Muskelzellen und in Neointimazellen. In kultivierten Zellen findet sich VASP assoziiert mit Zell-Matrix-Kontaktstellen (fokalen Adhäsionspunkten), Zell-Zell Kontakten, dem Aktinfilamentsystem und dynamischen Membranstrukturen, z.B. dem Leitsaum beweglicher Zellen. Viele experimentelle Daten belegen, daß VASP als Adaptermolekül Profilaktin an Orten mit den Zytoskelettproteinen Zyxin und Vinculin oder mit dem Oberflächenprotein ActA in mit Listeria spec. infizierten Zellen bereitstellt. Das EVH1-Domänen bindende FPPPP-Motiv in den Proteinen Zyxin, Vinculin und ActA und die EVH1-Domäne in VASP wurden funktionell und auch strukturell mittels NMR-Strukturaufklärung charakterisiert. Funktionelle Untersuchungen belegen, daß VASP ein entscheidender Faktor für eine gesteigerte, ortsgebundene Aktinfilamentbildung und damit auch ein wichtiger Faktor für die Regulation von Zelladhäsion und Zellmotilität ist. VASP wirkt dabei in direkter Wechselwirkung mit anderen Proteinen wie beispielsweise Zyxin, Vinculin oder Profilin. Dies konnte z.B. durch die Mikroinjektion von Peptiden, welche das Bindungsmotiv der VASP-Zyxin-Wechselwirkung enthalten, gezeigt werden. [Eine Übersicht hierzu in: Reinhard M, Jarchau T, Reinhard K, and Walter U. (1999) VASP. In : Guidebook to the Cytoskeletal and Motor Proteins (Eds., Kreis,T., and Vale,R.), Oxford University Press, Oxford, pp. 168 -171]. Aus diesen Gründen wird der Komplex zwischen VASP und Zyxin als neuartige potentielle Zielstruktur angesehen, um durch Entwicklung entsprechender diese Wechselwirkung modulierender Arzneimittel Erkrankungen mit pathologisch veränderter Zelladhäsion und Zellmotilität wie beispielsweise Arterienverkalkung, Gerinnungsstörungen und damit im Zusammenhang stehende Herz-Kreislauferkrankungen günstig beeinflußen zu können. VASP und Zyxin bzw. Homologe oder Derivate dieser Proteine, welche miteinander wechselwirken, können deshalb unter anderem dazu verwendet werden, chemische Substanzen zu identifizieren, die als therapeutische Wirkstoffe zur Behandlung von Herz-Kreislauferkrankungen eingesetzt werden können.

Bekannt ist eine Methode zum Nachweis der Wechselwirkung zwischen einer EVH1-Bindedomäne wie beispielsweise in Zyxin enthalten mit einer EVH1-Domäne wie beispielsweise in VASP enthalten als radioaktiver Festphasen-Assay oder Overlay-Assay, wobei die Detektion von Zyxin nach Übertragung auf eine Festphase mit oder ohne vorherige gelelektrophoretische Auftrennung durch radioaktiv markiertes VASP erfolgt. (Reinhard et al. PNAS 92, 7956 - 7960, 1995; Reinhard et al. FEBS Lett. 399, 103 - 107, 1996). Dieses Verfahren ist aufgrund seines Formats und seiner radioaktiven Detektion nicht für ein Nachweisverfahren mit hohem Probendurchsatz (High-Througput Screening = HTS) geeignet. Die verwendete radioaktive Markierungsmethode beschränkt die Anwendungsbreite, eine gelelektrophoretische Auftrennung ermöglicht wegen der aufwendigen Trennschritte keine Verwendung in automatisierten Screeningverfahren und erzeugt darüberhinaus Probleme hinsichtlich der Spezifität des Nachweises.

In WO 98/01755 werden dem VASP ähnliche Proteine (Mena, Evl) offenbart, die nicht aus Menschen stammen. Diese Proteine sind für den Aufbau eines Screeningmodells weniger geeignet, da sie die nachzubildende Zielstruktur aus vorzugsweise humanen Komponenten für pharmazeutische Screeningzwecke nur unvollkommen wiedergeben.

Die Verwendung von Lanthanid-Chelaten als Fluoreszenzmarkierung und die Verwendung zeitaufgelöster Fluoreszenzmessung mittels dieser Fluoreszenzmarkierungen für beispielsweise HTS (High Througput Screening) wird von Wallac Oy (Finnland) in WO 97/29373 und WO 98/15830 offenbart. Es liegen bislang keine publizierten Ergebnisse vor zur Benutzung von Fluoreszenzmarkierungen für die Analyse der Wechselwirkung zwischen einer EVH1-Bindedomäne wie beispielsweise in Zyxin enthalten mit einer EVH1-Domäne wie beispielsweise in VASP enthalten, die insbesondere für eine Verwendung in Screeningverfahren geeignet wären.

WO 01/96594 A gilt gemäß Art. 54(3) EPÜ als Stand der Technik. Diese ältere Anmeldung zeigt ein HTS-geeignetes Testsystem zum Nachweis der Aktivität von VASP - Phosphatasen.

Aus AHERN-DJAMALI S M ET AL: "Mutations in Drosophila enabled and rescue by human vasodilator-stimulated phosphoprotein (VASP) indicate important functional roles for Ena/VASP homology domain 1 (EVH1) and EVH2 domains", MOLECULAR BIOLOGY OF THE CELL, BETHESDA, MD, US, Bd. 9, Nr. 8, 1. August 1998, Seiten 2157-2171, ISSN: 1059-1524, ist bekannt, dass Zyxin und VASP interagieren können.

Aus BALL L J ET AL: "Dual epitope recognition by the VASP EVH1 domain modulates polyproline ligand specificity and binding affinity", EMBO JOURNAL, OXFORD UNIVERSITY PRESS, SURREY, GB, Bd. 19, Nr. 18, 15. September 2000, Seiten 4903-4914, ISSN: 0261-4189, ist auch bekannt, dass Zyxin und VASP interagieren können. Es wurden Peptide mit der Sequenz "FPPPP" sowie "WPPPP" verwendet.

MACALMA TERESITA ET AL: "Molecular characterization of human zyxin", JOURNAL OF BIOLOGICAL CHEMISTRY, AMERICAN SOCIETY OF BIOLOCHEMICAL BIOLOGISTS, BIRMINGHAM, US, Bd. 271, Nr. 49, 1. Januar 1996, Seiten 31470-31478, ISSN: 0021-9258, beschreibt die molekulare Charakterisierung von humanem Zyxin.

Aus KRAUSE MATTHIAS ET AL: "Fyn-binding protein (Fyb)/SLP-76-associated protein (SLAP), Ena/vasodilator-stimulated phosphoprotein (VASP) proteins and the Arp2/3 complex link T cell receptor (TCR) signaling to the actin cytoskeleton", THE JOURNAL OF CELL BIOLOGY, ROCKEFELLER UNIVERSITY PRESS, US, Bd. 149, Nr. 1, 3., Seiten 181-194, ISSN: 0021-9525, ist auch bekannt, dass Zyxin und VASP interagieren können.

SMOLENSKI A ET AL: "Regulation of human endothelial cell focal adhesion sites and migration by cGMP-dependent protein kinase I", JOURNAL OF BIOLOGICAL CHEMISTRY, AMERICAN SOCIETY OF BIOLOCHEMICAL BIOLOGISTS, BIRMINGHAM, US, Bd. 275, Nr. 33, 20. August 2000, Seiten 25723-25732, ISSN: 0021-9258, offenbart die Verwendung von anti-VASP monoklonalen Antikörper.

Die Aufgabe wird daher in der Entwicklung eines Screeningverfahrens zur Identifizierung von chemischen Verbindungen gesehen, welche die Wechselwirkung zwischen eines Proteins mit einer EVH1-Bindedomäne mit eines Proteins mit einer EVHI-Domäne modifizieren können. Dieses Screeningverfahren sollte sich für hohen Durchsatz eignen sowie sicher, schnell, mit hoher Spezifität und zuverlässig auch im automatisierten Betrieb durchführbar sein. Darüberhinaus sollte in diesem Screeningverfahren eine für pharmazeutisches Screening relevante Zielstruktur mit vorzugsweise humanen Komponenten nachgebildet werden.

Ein Verfahren zur Identifizierung einer chemischen Verbindung, welche eine Wechselwirkung zwischen einem Protein mit einer EVH1-Bindedomäne und einem Protein mit einer EVH1-Domäne moduliert, wird beschrieben, enthaltend die Verfahrensschritte:
a) In-Kontakt-Bringen eines Proteins mit einer EVH1-Bindedomäne mit einem Protein mit einer EVH1-Domäne in Gegenwart einer zu untersuchenden chemischen Verbindung; Unter "In-Kontakt-Bringen" kann insbesondere die Abfolge der Schritte von Beschichten eines Trägers mit einer Domäne, Blocken/Waschen, Zugabe der anderen Dömäne (mit oder ohne Testsubstanz), Inkubation bis einschließlich Waschen verstanden werden;
b) Verwendung des Ansatzes gemäß a) zur Inkubation mit einem Antikörper, der Bindespezifität für ein Protein mit einer EVH1-Domäne oder für ein mit diesern Protein fusioniertes oder gekoppeltes Antigen hat;
c) Verwendung des Ansatzes gemäß b) zur Inkubation mit einem Antikörper, der den Antikörper aus Ansatz b) spezifisch binden kann und an dem eine biochemisch oder physikalisch-chemisch nachweisbare Markierung angebracht ist;
d) biochemischer oder physikalisch chemischer Nachweis der Markierung am Antikörper aus c) nach Inkubation gemäß c).

Die Modulation der Wechselwirkung kann zu einer Verstärkung der Bindung der Bindungspartner oder zu einer Abschwächung dieser Bindung führen. Die Verstärkung der Bindung der Bindungspartner zeigt sich beispielsweise an der Erhöhung der Affinität der beteiligten Domänen oder Proteine zueinander. Eine Erhöhung der Affinität gibt sich an der Erniedrigung der Affinitätskonstanten der beteiligten Bindungspartner zu erkennen. Die Affinitätskonstanten sind mit Standardmethoden der Biochemie bestimmbar. Solche Methoden sind beispielsweise offenbart in "Pingoud, A., Urbanke, K., Arbeitsmethoden der Biochemie; 1997; Gruyter Lehrbuch" oder in "Wilson, K., Goulding, K. H., Methoden der Biochemie; 1991; Thieme flexible Taschenbücher". Das analoge gilt für die Abschwächung der Bindung der Bindungspartner. Ein Protein mit einer EVH1-Bindedomäne ist ein Protein, welches eine EVH1-Bindedomäne enthält. Ebenso ist ein Protein mit einer EVH1-Domäne ein Protein, welches eine EVH1-Domäne enthält.

Das Verfahren zur Identifizierung einer chemischen Verbindung wie vorstehend beschrieben erfolgt an einer Oberfläche, die aus einem festen Körper besteht. Es wird deshalb auch als Festphasen-Assay bezeichnet. Die Oberfläche des festen Körpers ist dabei mit einem Protein mit einer EVH1-Bindedomäne beschichtet, welche mit einem Protein mit einer EVH1-Domäne wechselwirkt. Die so beschichteten Oberflächen können dann mit einem bezüglich der untersuchten Wechselwirkung inerten Reagenz oder Protein wie Rinderserumalbumin beschichtet werden. Auf diese so beschichtete Oberfläche wird die zu untersuchende chemische Verbindung aufgegeben, wobei sie in einem wäßrigen oder organischen Lösungsmittel gelöst sein kann. Der feste Körper kann aus unterschiedlichem Material wie Kunststoff, Glas oder Metall bestehen. Bevorzugt besteht der feste Körper aus einem organischen Polymer. In einer Ausführungsform besteht der feste Körper aus einem chemischen Material , das unlöslich in oder widerstandsfähig gegen organische Lösungsmittel, Säuren, Laugen oder wässige Lösungen ist. Der feste Körper kann in unterschiedlicher Form aufgebaut sein. Beispielsweise kann er in einer bevorzugten Variante als Mikrotiterplatte sowie als Eppendorfgefäß, Glasröhrchen, Folie oder Mikrochip vorliegen. Der feste Körpers kann aus nur einem oder mehreren Materialien oder Komponenten bestehen. Die Beschichtung der Oberfläche des festen Körpers mit einem Protein mit einer EVH1-Bindedomäne, welche mit einem Protein mit einer EVH1-Domäne wechselwirkt, kann direkt am festen Körper durchgeführt werden. Es ist im Falle aus unterschiedlichen Materialien zusammengesetzter fester Körper auch möglich ein Trägermaterial separat von einem festen Grundkörper zu beschichten und das Trägermaterial nach der Beschichtung auf diesem festen Grundkörper aufzubringen, wobei dann das Trägermaterial und der feste Grundkörper zusammen den festen Körper bilden. Die Beschichtung der Oberfläche kann zuerst allein mit einem Protein mit einer EVH1-Bindedomäne erfolgen. Die Wechselwirkung mit einem Protein mit einer EVH1-Domäne wird dann hergestellt, indem in einem zweiten Schritt ein Protein mit einer EVH1-Domäne zu der mit einem Protein mit der EVH1-Bindedomäne beschichteten Oberfläche zugegeben wird. Zur Beschichtung der Oberfläche des festen Körpers mit einem Protein mit einer EVH1-Bindedomäne wird die Oberfläche des festen Körpers mit dem Protein inkubiert. Ebenso werden zur Herstellung der Wechselwirkung zwischen einem Protein mit einer EVH1-Bindedomäne und einem Protein mit einer EVH1-Domäne ein Protein mit dieser Domäne mit einem Protein mit dieser anderen Domäne inkubiert. Der Überschuß an nicht-wechselwirkenden Proteinen wird dann durch einen Waschschritt entfernt. Inkubieren bedeutet dabei, daß Proteine und Oberfläche über eine definierte Zeitspanne bei festgelegter Temperatur und bestimmten Puffer-und Ionenkonzentrationen miteinander in Kontakt gebracht werden. Die Proteine können dazu in gepufferten und mit chemischen Zusätzen versehenen wäßrigen Medien gelöst oder suspendiert vorliegen.

Als Protein mit einer EVH1-Bindedomäne wird humanem Zyxin (1-142) verwendet. Als Protein mit einer EVH1-Domäne wird humanem VASP eingesetzt.

Die benötigten Proteine können entweder aus entsprechenden Geweben oder Zellen von Wirbeltieren wie zum Beispiel Blutplättchen isoliert werden oder in Wirtszellen oder Mikroorganismen wie beispielsweise Insektenzellen oder E. coli Zellen rekombinant hergestellt und aufgereinigt werden. Rekombinantes VASP wird z.B. aus Insektenzellen mittels Immunoaffinitätschromatographie aufgereinigt wie detailliert beschrieben in "Jarchau, T., Mund, T., Reinhard, M., U. Walter (1998) Purification and Assays of Vasodilator-Stimulated Phosphoprotein. Methods in Enzymology Vol. 298, 103 - 113". Die EVH1-Domäne von VASP oder die EVH1-Bindedomäne von Zyxin werden z.B. rekombinant aus E.coli als Glutathion-S-Transferase -Fusionsproteine gereinigt wie in "Current Protocols in Molecular Biology, ed.: F.M. Ausubel, Wiley-Interscience (1987)" detailliert beschrieben.

Als Protein mit einer EVH1-Bindedomäne kann auch ein Zyxinderivat eingesetzt werden: Das Zyxinderivat besteht aus einem Fusionsprotein aus der Glutathion-S-Transferase, an deren C- Terminus die ersten 142 Aminosäuren des N-Terminus des Zyxin fusioniert worden sind. Als Glutathion-S-Transferase kann grundsätzlich die Aminosäuresequenz jeder Spezies verwendet werden. Sequenz von Mensch ist beispielsweise in Swissprot offenbart unter P08263 (Mensch)

Ein Verfahren zur Identifizierung einer chemischen Verbindung, welche eine Wechselwirkung zwischen einem Protein mit einer EVH1-Bindedomäne und einem Protein mit einer EVH1-Domäne modulieren, wird mittels einer Oberfläche durchgeführt, die aus einem festen Körper besteht, der mit Proteinen wie vorstehend beschrieben beschichtet ist, und den Teil einer Mikrotiterplatte bildet.

Zur Durchführung des Verfahrens zum spezifischen Nachweis einem Protein mit einer EVH1-Domäne können monoklonale oder polyklonale Antikörper verwendet werden. Diese monoklonalen oder polyklonalen Antikörper müssen Bindespezifität eine EVH1-Domäne, ein Protein mit einer EVH1-Domäne oder ein mit diesen Domänen oder Proteinen fusioniertes oder chemisch gekoppeltes Antigen aufweisen.

Zur Durchführung des Verfahrens wird beispielsweise ein monoklonaler Antikörper verwendet, der Bindespezifität für VASP hat. Solch ein Antikörper kann mittels Hybridomzellen synthetisiert und anschließend konzentriert und gereinigt werden. Die Kultivierung von Hybridomzellen, die Produktion von Antikörpern mit Hilfe von Hybridomzellen, deren Reinigung und Konzentrierung erfolgt nach Standardmethoden wie beispielsweise in "Current Protocols in Immunology, ed.: J. E. Coligan, Wiley-Interscience (1991)" beschrieben. Ebenso sind aus diesem Lehrbuch Verfahren zu entnehmen, die es ermöglichen, die Bindespezifität für ein Antigen zu bestimmen. Als Antigen kann eine EVH1-Domäne oder ein Protein mit einer EVH1-Domäne beispielsweise VASP oder ein mit diesen Domänen oder Proteinen fusioniertes Antigen wie beispielsweise Glutathion-S-Transferase, Hexahistidin, Thioredoxin oder Maltosebindeprotein oder ein chemisch gekoppeltes Antigen eingesetzt werden. Zur Durchführung des Verfahrens wird in einer Ausführungsform ein polyklonaler Antikörper verwendet, der Bindespezifität für eine EVHI-Domäne oder ein Protein mit einer EVH1-Domäne wie VASP oder ein mit diesen Domänen oder Proteinen fusioniertes Antigen wie beispielsweise Glutathion-S-Transferase, Hexahistidin, Thioredoxin oder Maltosebindeprotein oder ein chemisch gekoppeltes Antigen hat. Herstellung, Reinigung, Test und Verwendung polyklonaler Antikörper ist in "Current Protocols in Immunology, ed.: J. E. Coligan, Wiley-Interscience (1991)" detailliert beschrieben.

In einer bevorzugten Ausführungsform des Verfahrens wird als Antikörper mit Bindespezifität für VASP der monoklonale Antikörper mAB IE245 und in einer weiteren bevorzugten Ausführungsform der monoklonale Antikörper mAB IE273 verwendet.

Die Antikörper, welche Antikörper des Verfahrens mit Bindespezifität für eine EVH1-Domäne, ein Protein mit einer EVH1-Domäne oder ein mit dieser Domäne oder Protein fusioniertes oder chemisch gekoppeltes Antigen spezifisch binden, können mit einer biochemisch oder physikalisch chemisch nachweisbaren Markierung versehen sein. Eine biochemisch oder physikalisch chemisch nachweisbare Markierung ist beispielsweise ein Enzym, ein radioaktives Isotop oder eine Fluoreszenzmarkierung. In einer bevorzugten Ausführungsform wird als biochemisch oder physikalisch chemisch nachweisbare Markierung insbesondere alkalische Phosphatase oder β-Galaktosidase, in einer weiteren bevorzugten Ausführungsform wird als biochemisch oder physikalisch chemisch nachweisbare Markierung ein Isotop wie beispielsweise ein radioaktives Isotop und in einer weiteren bevorzugten Ausführungsform wird als biochemisch oder physikalisch chemisch nachweisbare Markierung eine Fluoreszenzmarkierung insbesondere ein Lanthanidkomplex wie ein Europium-Komplex verwendet.

Ein wie vorstehend beschriebenes Verfahren kann zur Identifizierung eines Arzneimittels verwendet werden. Solche Arzneimittel können unter anderem zur Behandlung von Herz-Kreislauferkrankungen, inflammatorischen Erkrankungen, Erkrankungen der Blutgefäße oder bei neoplastischen Zell- und Gewebsveränderungen wie beispielsweise Krebs verwendet werden.

Eine chemische Verbindung zur Modulierung der Wechselwirkung zwischen einer EVH1-Bindedomäne oder einem Protein mit einer EVH1-Bindedomäne und einer EVH1-Domäne oder einem Protein mit einer EVH1-Domäne, kann über ein Verfahren wie vorstehend beschrieben, identifiziert werden. Solche chemischen Verbindungen sind bevorzugt Peptide insbesondere mit den Sequenzen FPPPP oder WPPPP oder deren chemische Derivate und prolinreichen Homologe. Solche chemischen Verbindungen können beispielsweise Arzneimittel zur Behandlung von Herz- Kreislauferkrankungen, inflammatorischen Erkrankungen, Erkrankungen der Blutgefäße oder von neoplastischen Zell- und Gewebsveränderungen wie beispielsweise Krebs sein.

Der monoklonale Antikörper mAB IE245, der Bindespezifität für VASP hat, sowie Hybridomazellen, welche den monoklonalen Antikörper mAB IE245 produzieren können können verwendet werden, Die Hybridomazellen, welche den monoklonalen Antikörper mABIE245 produzieren können, bestehen aus dem Stamm DSM ACC2444. Der monoklonale Antikörper mAB IE273, der Bindespezifität für VASP hat, sowie auf Hybridomazellen, welche den monoklonalen Antikörper mAB IE273 produzieren können können verwendet werden. Die Hybridomazellen, welche den monoklonalen Antikörper mABIE273 produzieren können, bestehen aus dem Stamm DSM ACC2445.

Die Hybridomazellen DSM ACC2444 und DSM ACC2445 sind bei der Deutschen Stammsammlung für Mikroorganismen hinterlegt.

Eine Oberfläche, die aus einem festen Körper besteht, und mit einem Protein mit einer EVH1-Bindedomäne beschichtet ist, wird beschrieben.

Das Protein mit einer EVH1-Bindedomäne besteht dabei aus humanem Zyxin (1-142) oder aus einem Fusionsprotein aus humanem Zyxin (1-142) und einer Glutathion-S-Transferase.

Eine Mikrotiterplatte, welche eine Oberfläche enthält, die mit einem Protein mit einer EVH1-Bindedomäne und beschichtet ist wird beschrieben. Die Mikrotiterplatte kann dabei unterschiedlich viele Gefäße umfassen. Beispielsweise kann die Mikrotiterplatte 3, 6, 12, 24, 48, 96, 192, 384, 768, 1536, 3072 oder mehr Gefäße (wells) enthalten. In einer bevorzugten Ausführungsform enthält die Mikrotiterplatte 384 Gefäße, in einer besonders bevorzugten Ausführungsform enthält sie 768 Gefäße und in einer ganz besonders bevorzugten Ausführungsform enthält die Mikrotiterplatte 1536 Gefäße. Die Oberfläche kann darüberhinaus Bestandteil von anderen Geräten, Gefäßen oder Vorrichtungen wie beispielsweise von Eppendorfgefäßen, Röhrchen aus unterschiedlichem Material insbesondere Kunststoff oder Glas, von chipartigen Vorrichtungen oder anderen Geräten, Vorrichtungen oder Behältern sein.

Aminosäuresequenz für VASP ist offenbart bei Swissprot unter P50552 (Mensch) . Aminosäuresequenz für Zyxin ist offenbart bei Swissprot unter Q15942 (Mensch).

### Beispiele

### Beispiel 1: Bestimmung der Wechselwirkung von VASP und Zyxin oder einem Zyxinderivat mittels eines Festphasen-Assays (DELFIA)

Beim Festphasen-Assay wird ein Bindungspartner (VASP oder Zyxin) auf der Oberfläche einer Mikrotiterplatte (MTP) immobilisiert (=Beschichtung). Nach dem Blocken der ungesättigten Bindungsstellen auf der Plastikoberfläche wird mit dem jeweiligen anderen Bindungspartner inkubiert. In einem Waschschritt wird das überschüssige, nicht gebundene Protein nach der Inkubation entfernt. Durch geeignete Antikörper kann jetzt das spezifisch gebundene Protein detektiert und quantifiziert werden. Hierbei kann die Verwendung von Lanthanid-Chelaten als Fluoreszenz-Markierung für die Antikörper und die Messung der zeitaufgelösten Fluoreszenz erheblich zur Verbesserung des Signal-Rausch-Verhältnisses (S/N) beitragen.

Für den Festphasenassay wurden zuerst die besten Beschichtungs- und Inkubationsbedingungen charakterisiert. Es stellte sich heraus, daß sich die Zyxin-Komponente (GST-Zyxin(1-142) oder Zyxin(1-142)) zum Beschichten der Mikrotiterplatten (MTP) sehr gut eignet. Für die Detektion des spezifisch an das immobilisierte Zyxin gebundenen VASP eignen sich monoklonale Antikörper gegen VASP, welche ihrerseits mittels Lanthanid-markierten Antikörpern gegen Maus IgGs nachgewiesen werden können (Wallac: DELFIA anti-Mouse-Eu(N1)).

Das gegenwärtige Assay-Protokoll besteht aus 5 Inkubationschritten und 3 Waschschritten:
1. Inkubation der leeren MTPs mit GST-Zyxin(1-142) (Beschichtung)
2. Blocken mit BSA (Rinderserumalbumin)
3. Waschen mit PBS (Waschpuffer)
4. Inkubation mit VASP (Ligand) (mit und ohne Testsubstanzen)
5. Waschen mit PBS
6. Inkubation mit Detektionsmix: α-VASP (= monoklonaler anti-VASP Antikörper) + α-Maus-Eu (= Europium markierter anti-Maus Antikörper von Wallac)
7. Waschen mit DELFIA-Wash-Lösung
8. Freisetzung des gebundenen Lanthanidkomplexes und fluorimetrische Messung.

Bei der Wahl der Reagenzien/Proteine stellte sich heraus, daß das rekombinante humane VASP Protein mit der vollständigen Aminosäuresequenz gewonnen aus Insektenzellen (baculo VASP) mit signifikant besserem Signal-Rausch-Verhältnis (S/N) funktionierte als VASP oder VASP-Domänen, welche in in E. coli exprimiert und daraus gewonnen wurden.

### Beispiel 2: Eigenschaften des VASP Zyxin Festphasen (DELFIA) Assay:

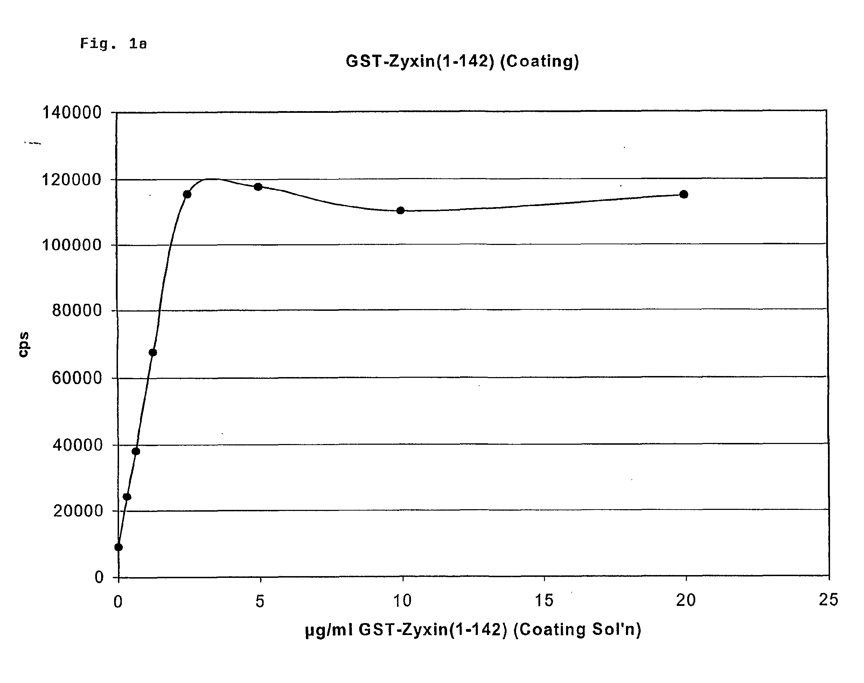

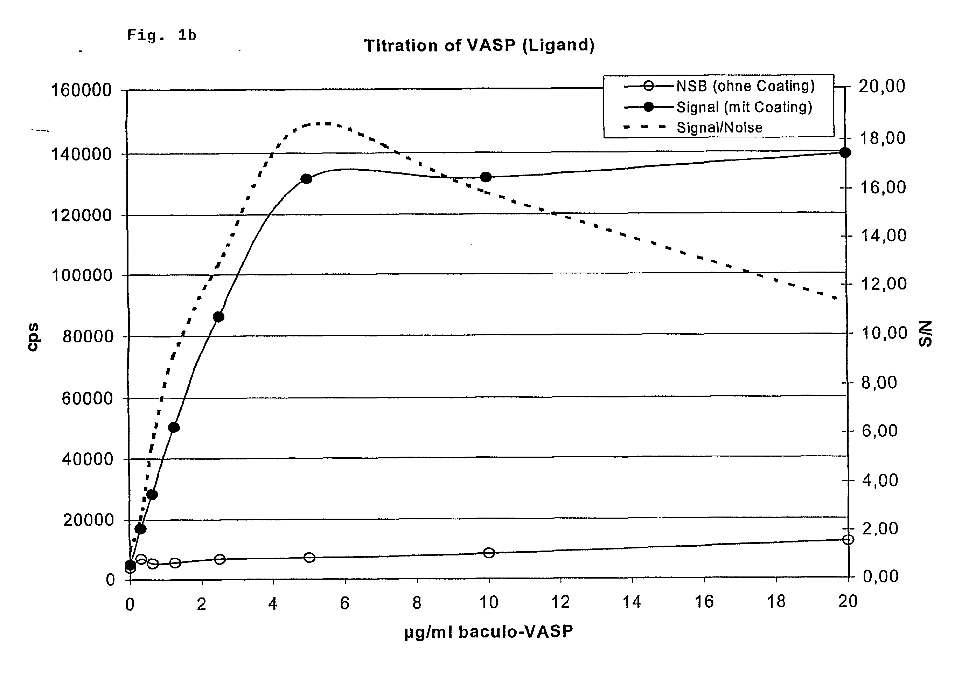

Die optimalen Bedingungen für Beschichtung (GST-Zyxin(1-142)) und Liganden-Inkubation (VASP) wurden durch Titration der Reagenzien ermittelt. Die Beschichtung der MTPs mit GST-Zyxin(1-142) ist bei einer Konzentration von 5 µg/ml der Beschichtungslösung in der Sättigung (Fig. 1 a). Das eingesetzte Volumen pro Well hängt vom gewählten MTP-Format ab (50 µl oder 100 µl bei 96-well Platten, 20 µl bei 384-well Platten). Für die Inkubation mit VASP als Ligand ergab sich ebenfalls eine Konzentration von 5 µg/ml als Wert mit dem besten S/N (Fig. 1 b).

### Beispiel 3: Detektion

Bei der weiteren Optimierung des Assays stellte man fest, daß die Wahl des monoklonalen Antikörpers für die Detektion des gebundenen VASPs von großer Bedeutung ist. Im Vergleich mit dem anti-VASP Antikörper mAB IE245 erreicht man mit dem Antikörper IE273 ein S/N, das um ca. eine Größenordnung besser ist. Die Optimierung der einzusetzenden Menge an IE273 Antikörper zeigte, daß diese abhängig von der vorher verwendeten Menge an VASP ist. Ein Optimum für S/N ergab sich für ein molares Verhältnis von IE273 zu VASP bei einem Wert von 1:16 massenmäßiges Verhältnis von 1:4). Bei Inkubation mit einer Konzentration von 5 µg/ml VASP ergab sich das beste Signal bei einer Detektion mit 1,25 µg/ml IE273. Dies ist auch von Bedeutung für die Optimierung bzw. Reduzierung der Waschschritte, da es zeigt, daß der Waschschritt zwischen Inkubation mit VASP und Detektion wichtig ist und nicht weggelassen werden darf.

### Beispiel 4: Kompetitionsversuche mit peptidergen Inhibitoren

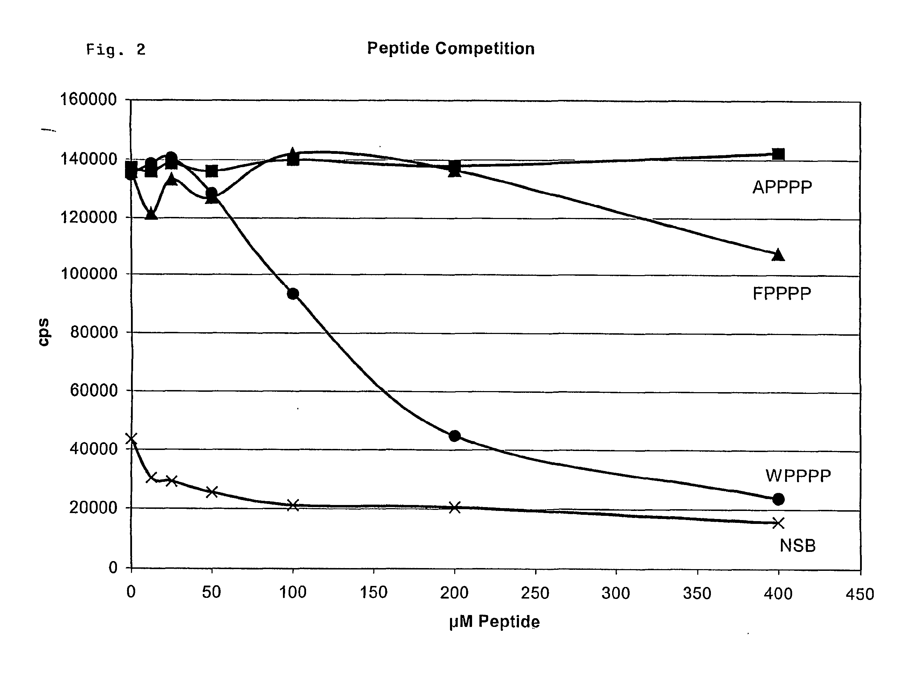

Für die VASP-Zyxin-Wechselwirkung existieren bislang noch keine bekannten nichtpeptidergen Inhibitoren. Um die Wirkung potenzieller Inhibitoren zu simulieren, wurden Inhibitionsversuche mit kompetierenden Peptiden unternommen, die das VASP-Bindungsmotiv von Zyxin (FPPPP) enthielten. In früheren Arbeiten (Niebuhr et al. (1997) EMBO J. 16, 5433) wurden Mutationen in diesem Motiv und ihre Wirkung auf die Inhibition der VASP-ActA-Wechselwirkung untersucht, die ebenfalls auf der Bindung von VASP an FPPPP-Motive in ActA beruht. Dabei wurde gezeigt, daß im Vergleich zur Wildtyp-Sequenz FPPPP Peptide mit einem APPPP-Motiv eine deutlich schlechtere Inhibition zeigten, wogegen Peptide mit einem WPPPP-Motiv sogar eine bessere Inhibition als das Wildtyp-Motiv aufwiesen (Inhibition: WPPPP > FPPPP > APPPP). Zyxinpeptide mit entsprechenden Mutationen wurden zur Kompetition der VASP-Zyxin-Wechselwirkung eingesetzt. Dabei ergab sich die gleiche Reihenfolge in bezug auf die apparenten Inhibitionskonstanten der Peptide wie für die VASP-ActA-Wechselwirkung (Fig. 2): WPPPP > FPPPP > APPPP.

Dies zeigt, daß es möglich ist, durch kompetierende Substanzen, und möglicherweise auch durch anders geartete Inhibitoren, die VASP-Zyxin Wechselwirkung zu beeinflussen und dies durch den beschriebenen Assay zu detektieren.

### Beispiel 5: Lösungsmittel Toleranzen

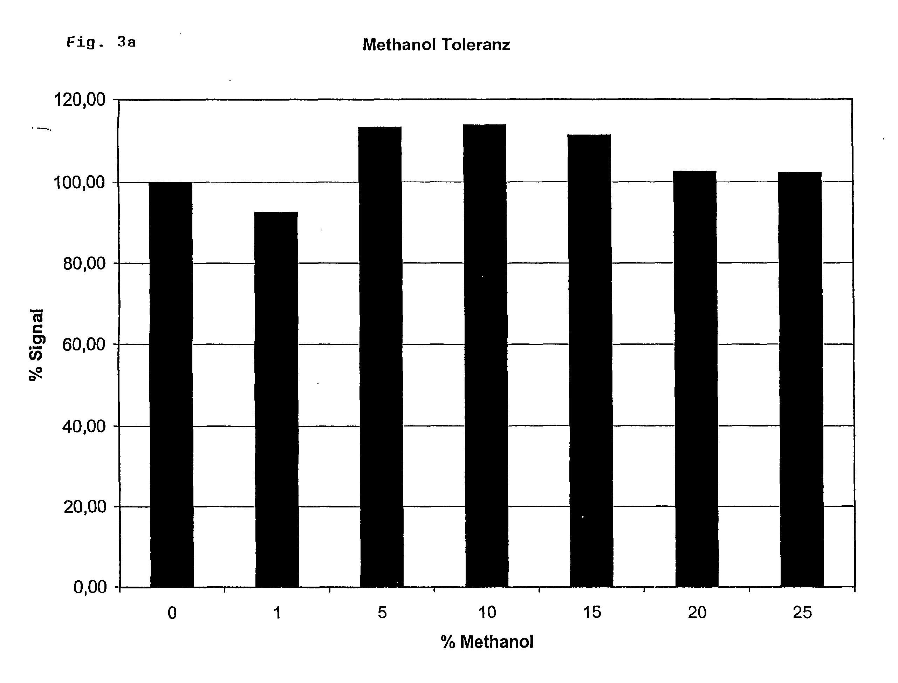

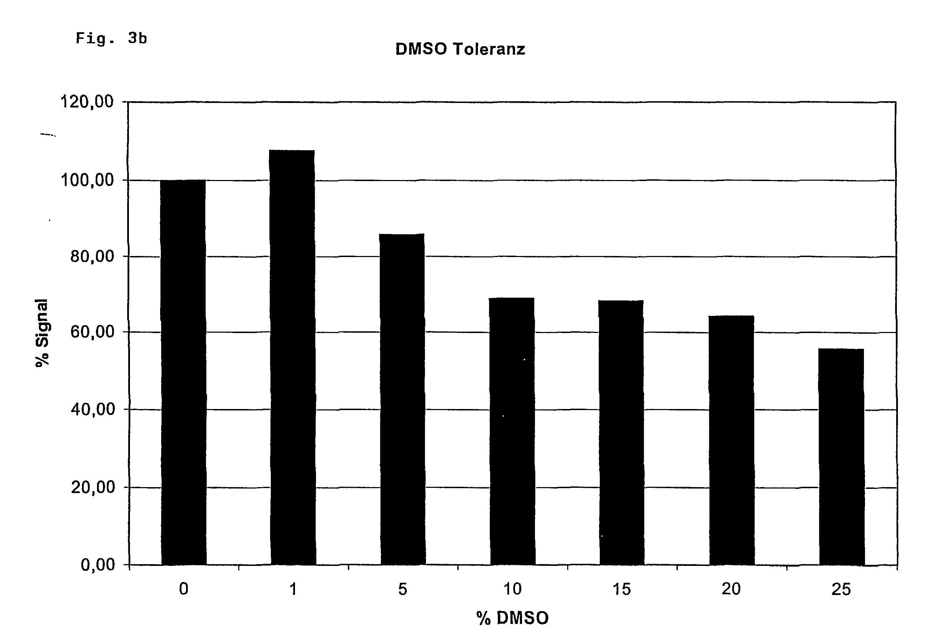

Um den Einsatz des Assays im HTS zu gewährleisten, muß sichergestellt sein, daß der Assay über eine ausreichende Toleranz gegenüber den Standardlösungsmitteln der Substanzbibliotheken verfügt. In der Regel sind diese Dimethylsulfoxid (DMSO) und Methanol. Um die Toleranz des Assays gegenüber diesen beiden Lösungsmitteln zu testen, wurde bei konstanter Beschichtung und konstanter Ligandenkonzentration (VASP) mit steigenden Konzentrationen Methanol und DMSO inkubiert. Es zeigte sich, daß Methanol bis zu einer Konzentration von 25% praktisch keinen Einfluß auf den Assay hat (Fig. 3a), während das Signal bei 25% DMSO fast um die Hälfte reduziert wurde (Fig. 3b). Bei im Screening gebräuchlichen DMSO-Konzentrationen von 1-5% betrug die Verringerung des Signals jedoch nur bis zu 15%, was im Rahmen eines HTS-Assay tragbar ist.

### Beispiel 6: Miniaturisierung

In der oben beschriebenen Weise wurden die Assays zunächst in 96-well Platten durchgeführt. Dabei konnte das Assayvolumen von 100 µl pro Well auf 50 µl reduziert werden. Zur weiteren Miniaturisierung des Assays für den Einsatz im HTS wurde der Assay auf 384-well Platten angepaßt. Dazu wurden 384-well Platten verschiedener Hersteller mit unterschiedlichen Oberflächen und Farben getestet. Unter den getesteten Platten ergaben sich die besten Werte für die 384-well Platten von Greiner. Dabei verhielten sich die weißen und schwarzen Platten sehr ähnlich, auch wenn die schwarzen Platten einen besonders niedrigen Hintergrund hatten. Unter den getesteten Oberflächen (low binding, untreated, high-bindung) wurden die besten Ergebnisse mit den unbehandelten Oberflächen (untreated) erzielt.

Durch die Verwendung von 384-well Platten konnte das Assayvolumen weiter auf 20 µl pro Well reduziert werden. Erfreulicherweise ergab sich als zusätzlicher Effekt der Miniaturisierung eine weitere Verbesserung des S/N auf ca. 100.

### Beispiel 7: Übersicht: Assay Protokoll (für 384-well Platten)

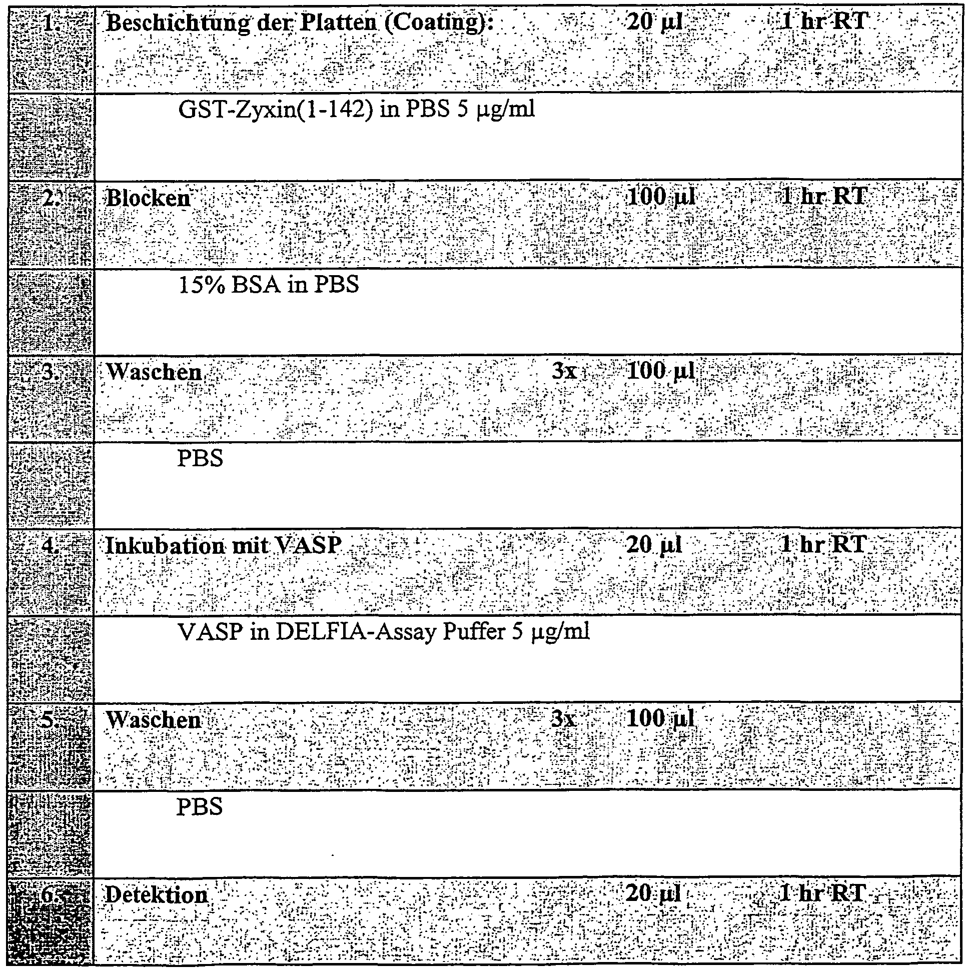

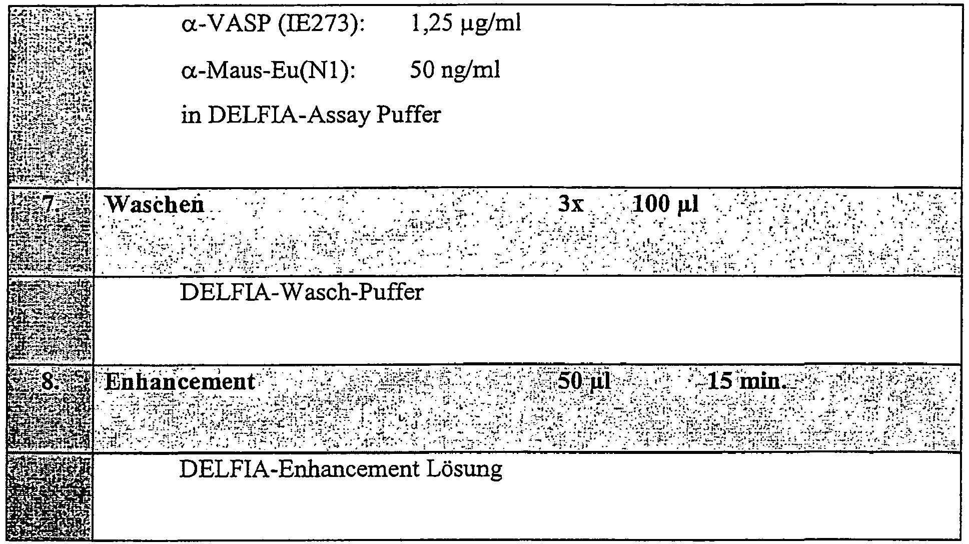

### Beispiel 8: Homogener Assay mittels Energietransfer bei fluoreszenzmarkiertem VASP und Zyxin oder einem Zyxinderivat:

Für den homogenen Assay wurde humanes VASP mit vollständiger Aminosäuresequenz , isoliert aus Insektenzellen (SF21), eingesetzt und über spezifische Antikörper indirekt mit einer Fluoreszenzmarkierung versehen. Als Bindungspartner wurden zunächst Peptide mit dem VASP-Bindungsmotiv FPPPP verwendet, die über ein gekoppeltes Biotin ebenfalls an den anderen Fluorophor für den Energietransfer (Streptavidin-APC) binden konnten. In folgenden Experimenten wurde dann jedoch dazu übergegangen, anstelle von Peptiden ein GST-Fusionsprotein mit dem N-Terminus von Zyxin (GST-Zyxin(1-142)), welches mehrere dieser FPPPP-Motive enthält, als Bindungpartner für VASP zu verwenden. Hierzu wurde GST-Zyxin(1-142) in E. coli exprimiert, aufgereinigt und mit Biotinen kovalent modifiziert. Damit kann ein Energietransfer zwischen den fluoreszenzmarkierten Bindungspartnern gemessen werden.

### Beispiel 9: Beschreibung der Abbildungen

Fig. 1a: Beschichtung der MTPs mit GST-Zyxin(1-142): Bei einer Konzentration von 5 µg/ml der Beschichtungslösung ist Sättigung erreicht.
Fig. 1b: Beschichtung der MTPs mit VASP: Für die Inkubation mit VASP als Ligand ergab sich eine Konzentration von 5 µg/ml als Wert mit dem besten S/N.
Fig.2: Inhibition der VASP-Zyxin-Wechselwirkung durch kompetierende Peptide, die das APPPP-, FPPPP-, oder WPPPP-Motiv enthalten.
Fig. 3a: Feststellung der Toleranz gegen Methanol; Inkubation mit steigenden Konzentrationen von Methanol bei konstanter Beschichtung und konstanter Ligandenkonzentration (VASP); Methanol zeigt bis zu einer Konzentration von 25% praktisch keinen Einfluß auf den Assay.
Fig. 3b: Feststellung der Toleranz gegen DMSO; Inkubation mit steigenden Konzentrationen von DMSO bei konstanter Beschichtung und konstanter Ligandenkonzentration (VASP); DMSO bewirkt bei 25% eine Reduktion des Signals um annähernd die Hälfte.

## Patentansprüche

1. In vitro Verfahren zur Identifizierung einer chemischen Verbindung, welche eine Wechselwirkung zwischen Zyxin und VASP moduliert, enthaltend die Verfahrensschritte:
a) In-Kontakt-Bringen von humanem Zyxin (1-142) oder einem Fusionsprotein, welches aus humanem Zyxin (1-142) und einer Glutathion-S-Transferase besteht, mit humanem VASP und mit einer zu untersuchenden chemischen Verbindung;
wobei das In-Kontakt-Bringen mit einer chemischen Verbindung an einer Oberfläche erfolgt, die aus einem festen Körper besteht und mit humanem Zyxin (1-142) oder einem Fusionsprotein, welches aus humanem Zyxin (1-142) und einer Glutathion-S-Transferase besteht, beschichtet ist;
b) Verwendung des Ansatzes gemäß a) zur Inkubation mit einem Antikörper, der Bindespezifitat für humanes VASP oder ein mit humanem VASP fusioniertes oder chemisch gekoppeltes Antigen hat;
c) Verwendung des Ansatzes gemäß b) zur Inkubation mit einem Antikörper, der den Antikörper aus Ansatz b) spezifisch binden kann und an dem eine biochemisch oder physikalisch chemisch nachweisbare Markierung angebracht ist;
d) biochemischer oder physikalisch chemischer Nachweis der Markierung am Antikörper aus c) nach Inkubation gemäß c).

2. Verfahren nach Anspruch 1, wobei die Oberfläche, die aus einem festen Körper besteht, den Teil einer Mikrotiterplatte bildet.

3. Verfahren nach Anspruch 1 oder 2, wobei zur Inkubation gemäß Verfahrensschritt b) ein polyklonaler Antikörper verwendet wird.

4. Verfahren nach einem oder mehreren der Ansprüche 1 bis 3, wobei zur Inkubation gemäß Verfahrensschritt b) ein monoklonaler Antikörper verwendet wird, der mittels Hybridomazellen synthetisiert wird.

5. Verfahren nach Anspruch 4, wobei als monoklonaler Antikörper mAB IE245 verwendet wird, der durch die Hybridomzellen DSM ACC2444 produziert wird.

6. Verfahren nach Anspruch 4, wobei als monoklonaler Antikörper mAB IE273 verwendet wird, der durch die Hybridomzellen DSM ACC2445 produziert wird.

7. Verfahren nach einem oder mehreren der Ansprüche 1 bis 6, wobei zur Inkubation gemäß Verfahrensschritt c) ein Antikörper verwendet wird, bei dem die biochemisch oder physikalisch chemisch nachweisbare Markierung ein radioaktives Isotop, ein Fluoreszenzfarbstoff oder ein Enzym ist.

8. Verfahren nach Anspruch 7, wobei ein Antikörper verwendet wird, bei dem das Enzym eine alkalische Phosphatase oder β-Galaktosidase ist.

9. Verfahren nach Anspruch 7, wobei ein Antikörper verwendet wird, bei dem der Fluoreszenzfarbstoff ein Lanthanid-Komplex ist.

10. Verfahren nach Anspruch 7, wobei der Lanthanid-Komplex ein Europium-Komplex ist.

11. In vitro Verfahren gemäß einem oder mehreren der Ansprüche 1 bis 10 zur Identifizierung eines Arzneimittels.

12. Oberfläche, die aus einem festen Körper besteht und mit humanem Zyxin (1-142) oder einem Fusionsprotein, welches aus humanem Zyxin (1-142) und einer Glutathion-S-Transferase besteht, beschichtet ist.

13. Oberfläche nach Anspruch 12, wobei das humane Zyxin (1-142) oder das Fusionsprotein, welches aus humanem Zyxin (1-142) und einer Glutathion-S-Transferase besteht, mit humanem VASP wechselwirkt.

14. Mikrotiterplatte, welche eine Oberfläche gemäß Anspruch 12 oder 13 enthält.

## Claims

1. An in vitro process for identifying a chemical compound which modulates an interaction between zyxin and VASP, which process comprises the following process steps:
a) bringing human zyxin (1-142) or with a fusion protein which consists of human zyxin (1-142) and a glutathione S-transferase into contact with human VASP and with a chemical compound to be examined;
where the bringing into contact with a chemical compound takes place at a surface which consists of a solid body and which is coated with human zyxin (1-142) or with a fusion protein which consists of human zyxin (1-142) and a glutathione S-transferase;
b) using the mixture according to a) for incubation with an antibody which specifically binds to human VASP or to an antigen which is fused with or chemically coupled to human VASP;
c) use of the mixture according to b) for incubation with an antibody which is capable of specifically binding the antibody from mixture b) and to which a label is attached which can be detected biochemically or physicochemically;
d) biochemical or physicochemical detection of the label on the antibody from c) after incubation according to c).

2. The process as claimed in claim 1, wherein the surface which consists of a solid body forms part of a microtiter plate.

3. The process as claimed in claim 1 or 2, wherein a polyclonal antibody is used for the incubation according to process step b).

4. The process as claimed in one or more of claims 1 to 3, wherein a monoclonal antibody which is synthesized using hybridoma cells is used for the incubation according to process step b).

5. The process as claimed in claim 4, wherein the monoclonal antibody used is mAB IE245 which is produced by the hybridoma cells DSM ACC2444.

6. The process as claimed in claim 4, wherein the monoclonal antibody used is mAB IE273 which is produced by the hybridoma cells DSM ACC2445.

7. The process as claimed in one or more of claims 1 to 6, wherein an antibody in which the biochemically or physiochemically detectable label is a radioactive isotope, a fluorescent dye or an enzyme is used for the incubation according to process step c).

8. The process as claimed in claim 7, wherein an antibody is used in which the enzyme is an alkaline phosphatase or β-galactosidase.

9. The process as claimed in claim 7, wherein an antibody is used in which the fluorescent dye is a lanthanide complex.

10. The process as claimed in claim 7, wherein the lanthanide complex is a europium complex.

11. The in vitro process as claimed in one or more of claims 1 to 10 for identifying a medicament.

12. A surface which consists of a solid body and which is coated with human zyxin (1-142) or with a fusion protein which consists of human zyxin (1-142) and a glutathione S-transferase.

13. The surface as claimed in claim 12, wherein the human zyxin (1-142) or the fusion protein which consists of human zyxin (1-142) and a glutathione S-transferase interacts with human VASP.

14. A microtiter plate which contains a surface as claimed in claim 12 or 13.

## Revendications

1. Procédé in vitro pour l'identification d'un composé chimique modulant une interaction entre la zyxine et la protéine VASP, qui comporte les étapes suivantes de processus :
a) mise en contact de zyxine humaine (1-142) ou d'une protéine de fusion qui consiste en zyxine humaine (1-142) et en une glutathion-S-transférase, avec de la VASP humaine et avec un composé chimique à étudier ;
la mise en contact avec un composé chimique s'effectuant sur une surface qui consiste en un corps solide et est revêtue avec de la zyxine humaine (1-142) ou une protéine de fusion qui consiste en zyxine humaine (1-142) et en une glutathion-S-transférase ;
b) utilisation de la préparation selon a) pour l'incubation avec un anticorps qui a une spécificité de liaison pour la protéine VASP humaine ou pour un antigène couplé chimiquement ou fusionné avec VASP humaine ;
c) utilisation de la préparation selon b) pour l'incubation avec un anticorps qui peut se lier spécifiquement à l'anticorps de la préparation b) et sur lequel est appliqué un marqueur détectable biochimiquement ou physico-chimiquement;
d) détection biochimique ou physico-chimique du marqueur sur l'anticorps de c) après incubation selon c).

2. Procédé selon la revendication 1, dans lequel la surface qui consiste en un corps solide fait partie d'une plaque de microtitrage.

3. Procédé selon la revendication 1 ou 2, dans lequel pour l'incubation selon l'étape b) du procédé on utilise un anticorps monoclonal.

4. Procédé selon une ou plusieurs des revendications 1 à 3, dans lequel pour l'incubation selon l'étape b) du procédé on utilise un anticorps monoclonal qui est synthétisé par des hybridomes.

5. Procédé selon la revendication 4, dans lequel on utilise comme anticorps monoclonal mAB IE245 qui est produit par la lignée d'hybridomes DSM ACC2444.

6. Procédé selon la revendication 4, dans lequel on utilise comme anticorps monoclonal mAB IE273 qui est produit par la lignée d'hybridomes DSM ACC2445.

7. Procédé selon une ou plusieurs des revendications 1 à 6, dans lequel pour l'incubation selon l'étape c) du procédé on utilise un anticorps dont le marqueur détectable biochimiquement ou physico-chimiquement est un isotope radioactif, un colorant fluorescent ou une enzyme.

8. Procédé selon la revendication 7, dans lequel l'enzyme est une phosphatase alcaline ou la β-galactosidase.

9. Procédé selon la revendication 7, dans lequel on utilise un anticorps dont le colorant fluorescent est un complexe de lanthanide.

10. Procédé selon la revendication 7, dans lequel le complexe de lanthanide est un complexe d'europium.

11. Procédé in vitro selon une ou plusieurs des revendications 1 à 10, pour l'identification d'un médicament.

12. Surface, qui consiste en un corps solide et est revêtue avec de la zyxine humaine (1-142) ou une protéine de fusion qui consiste en zyxine humaine (1-142) et en une glutathion-S-transférase.

13. Surface selon la revendication 12, sur laquelle la zyxine humaine (1-142) ou la protéine de fusion, qui consiste en zyxine humaine (1-142) et en une glutathion-S-transférase, interagit avec la protéine VASP humaine.

14. Plaque de microtitrage, qui comporte une surface selon la revendication 12 ou 13.
